# EUROPEAN PATENT APPLICATION

(11) **EP 4 656 179 A1**
(43) Date of publication of application: **03.12.2025**
(21) Application number: 23918738.8
(22) Date of filing: 19.10.2023
(51) Int. Cl.: A61K 8/99, A61K 8/64, A61Q 19/00, A61K 35/744, A61K 38/12, A61P 17/00, A61P 17/08, A61P 17/10, A61P 17/04, C12N 1/20

(54) **COMPOSITION CONTAINING STREPTOCOCCUS SP. STRAIN FOR PREVENTION, ALLEVIATION, OR TREATMENT OF SKIN DISEASES**

(30) Priority: 25.01.2023 KR 20230009298; 25.08.2023 KR 20230111978
(71) Applicant: Cosmax, Inc., Hwaseong-si, Gyeonggi-do 18622 (KR)
(72) Inventor: HEO, Young Mok, Seongnam-si, Gyeonggi-do 13486 (KR); LEE, Dong Geol, Seongnam-si, Gyeonggi-do 13486 (KR); KANG, Seung Hyun, Seongnam-si, Gyeonggi-do 13486 (KR); PARK, Myeong Sam, Seongnam-si, Gyeonggi-do 13486 (KR)
(74) Representative: Lavoix
(86) International application number: PCT/KR2023/016287
(87) International publication number: WO 2024/158103

(57) **Abstract**

The present invention provides a composition for the prevention, alleviation, or treatment of skin disease, comprising three Streptococcus spp. strains, cultures, lysates, extracts, or fermentation products thereof, or a core efficacy substance thereof, Cyclo(Gly-Pro). The composition may be advantageously utilized for the prevention, alleviation, or treatment of atopic dermatitis, or the like.

## Description

### Technical Field

The present invention relates to a composition for prevention, alleviation, or treatment of skin disease, including a Streptococcus sp. strain, a culture, a lysate, an extract, or a fermentation product thereof, or an ingredient derived therefrom.

### Background Art

The skin ecosystem provides microorganisms with many different types of habitats and is home to a wide variety of microorganisms. Humans, as hosts, have a symbiotic relationship with these microorganisms, which are known to have many positive effects on the hosts. The skin contains many different types of habitats, including invaginations and specialized niches, and supports a wide range of microorganisms to grow. Basically, the skin forms a physical barrier and helps defend against potential hazards and toxins from the outside. The skin is the point of contact with the external environment and is also a gathering place for a wide variety of microorganisms (fungi, bacteria, viruses, and small larvae). Microorganisms create habitats by adapting to specialized niches according to a selection of physical and chemical functions. Many studies have shown that such resident skin flora is involved in immune learning and inflammatory response regulation of the skin, and thus are attracting attention as a research subject and a potential solution for improving and treating inflammatory skin disease.

The skin often comes into direct contact with external stimuli such as UV rays and various pathogens, and is subject to strong influences from within the body. In addition, the skin is also affected by pathological changes such as genetics, inflammation, benign and malignant tumors, hormones, trauma, and degenerative changes, and thus, skin disease can be caused by various causes. Inflammatory dermatoses refer to conditions in which a series of clinical signs and symptoms, such as itching, edema, erythema, and peeling, are caused by a variety of irritants that trigger a series of inflammatory reactions within the skin epithelium, and known examples include atopic dermatitis, contact dermatitis, seborrheic dermatitis, and acne. Until now, the treatment of inflammatory dermatoses such as those described above has mainly involved antihistamines, vitamin ointments, or corticosteroids. However, the effects of these medications are often temporary and the side effects are often severe.

Accordingly, there is a need to develop materials of skin microbiota that can be useful in improving skin disease such as atopic dermatitis.

### Disclosure of Invention

### Technical Goal of the Invention

Accordingly, the inventors of the present invention have identified a Streptococcus sp. strain, a culture, a lysate, an extract, or a fermented product thereof, or a core efficacy substance thereof, Cyclo(Gly-Pro), for the prevention, alleviation, and treatment of skin disease.

One aspect is to provide a cosmetic composition for prevention or alleviation of skin disease, the cosmetic composition including three Streptococcus spp. strains, cultures, lysates, extracts, or fermented products thereof.

Another aspect is to provide a cosmetic composition for prevention or alleviation of skin disease, the cosmetic composition including Cyclo(Gly-Pro).

Another aspect is to provide a pharmaceutical composition for prevention or treatment of skin disease, the pharmaceutical composition including three Streptococcus spp. strains, cultures, lysates, extracts, or fermented products thereof.

Another aspect is to provide a pharmaceutical composition for prevention or treatment of skin disease, the pharmaceutical composition including Cyclo(Gly-Pro).

Another aspect is to provide a method of preventing, alleviating or treating skin disease, the method including administering an effective amount of the composition to a subject in need thereof.

### Means for Achieving Technical Goal

One aspect provides a cosmetic composition for prevention or alleviation of skin disease, the cosmetic composition including three Streptococcus spp. strains, cultures, lysates, extracts, or fermented products thereof.

Specifically, one aspect provides a cosmetic composition for prevention or alleviation of skin disease, the cosmetic composition including a mixture of (1), (2), and (3) below:
(1) a *Streptococcus pneumoniae* strain, a culture, a lysate, an extract, or a fermentation product thereof;
(2) a *Streptococcus infantis* strain, a culture, a lysate, an extract, or a fermentation product thereof; and
(3) a *Streptococcus mitis* strain, a culture, a lysate, an extract, or a fermentation product thereof.

The *S. pneumoniae* strain may include 16S rRNA of SEQ ID NO: 1. In an embodiment, the *S. pneumoniae* strain may include 16S rRNA having a sequence identity of at least about 95 %, at least about 96 %, at least about 97 %, at least about 98 %, at least about 99 %, at least about 99.5 %, at least about 99.9 %, 95 % to 100 %, 96 % to 100 %, 97 % to 100 %, 98 % to 100 %, 99 % to 100 %, 99.5 % to 100 %, or 99.9 % to 100 %, with SEQ ID NO: 1.

The *S. pneumoniae* strain may be a *Streptococcus* CX-2 strain deposited under accession number KCCM12005P.

The *S. infantis* strain may include 16S rRNA of SEQ ID NO: 2. In an embodiment, the *S. infantis* strain may include 16S rRNA having a sequence identity of at least about 95 %, at least about 96 %, at least about 97 %, at least about 98 %, at least about 99 %, at least about 99.5 %, at least about 99.9 %, 95 % to 100 %, 96 % to 100 %, 97 % to 100 %, 98 % to 100 %, 99 % to 100 %, 99.5 % to 100 %, or 99.9 % to 100 %, with SEQ ID NO: 2.

The *S. infantis* strain may be *S. infantis* CX-4 strain deposited under accession number KCCM12642P.

The *S. mitis* strain may include 16S rRNA of SEQ ID NO: 3. In an embodiment, the *S. mitis* strain may include 16S rRNA having a sequence identity of at least about 95 %, at least about 96 %, at least about 97 %, at least about 98 %, at least about 99 %, at least about 99.5 %, at least about 99.9 %, 95 % to 100 %, 96 % to 100 %, 97 % to 100 %, 98 % to 100 %, 99 % to 100 %, 99.5 % to 100 %, or 99.9 % to 100 %, with SEQ ID NO: 3.

The *S. mitis* strain may be *S. mitis* CX-8 strain deposited under accession number KCCM12656P.

In the present specification, the term "mixture of (1), (2), and (3)" may be used interchangeably with "a mixture of three Streptococcus spp. strains". The mixture of three Streptococcus spp. strains may refer to not only a mixture of the strains themselves, but also a mixture of strain-derived substances such as cultures of the strains, lysates of the strains, extracts of the strains, or fermented products of the strains.

The mixture of three Streptococcus spp. strains may exhibit synergistic effects on the prevention, alleviation, or treatment of skin disease compared to the strains used individually. In particular, the S. *infantis* strain (e.g., CX-4 strain) may play a major role in synergistic effects on the prevention, alleviation, or treatment of skin disease.

In an embodiment, as a result of evaluating the efficacy of reducing thymic stromal lymphopoietin (TSLP) expression, it was confirmed that a mixture of a culture filtrate of *S. pneumoniae* CX-2 and a culture filtrate of S. *mitis* CX-8 showed no synergistic effect, whereas a mixture of three strains including a culture filtrate of S. *infantis* CX-4 showed a synergistic effect.

The mixture of three Streptococcus spp. strains may improve skin barrier functions and relieve itchiness.

The mixture of three Streptococcus spp. strains may increase the relative abundance of beneficial skin bacteria and suppress microorganisms causing atopy, thereby improving and normalizing the skin microbiome.

The mixture of three Streptococcus spp. strains may inhibit or reduce expression of inflammatory markers, and increase expression of filaggrin.

Specifically, the mixture of three Streptococcus spp. strains may have one or more of the following effects:
(i) an effect of reducing expression of thymic stromal lymphopoietin (TSLP);
(ii) an effect of reducing the Investigator's Global Assessment (IGA) score;
(iii) an effect of reducing the Eczema Area and Severity Index (EASI) score;
(iv) an effect of increasing skin moisture content;
(v) an effect of reducing transepidermal water loss content;
(vi) an effect of reducing mild or worse itchiness;
(vii) an effect of reducing sleep disturbance of any severity caused by itching;
(viii) an effect of increasing relative abundance of beneficial skin bacteria (e.g., microorganisms of the genus Streptococcus);
(ix) an effect of suppressing *Staphylococcus aureus* which is an atopy-causing microorganism;
(x) an effect of reducing expression of any one or more of CCL17, CCL22, IL-13, IL-22, sIL-2R, IL-31; and
(xi) an effect of increasing expression of filaggrin (FLG).

The mixture may include Cyclo(Gly-Pro) derived from the aforementioned strains. The Cyclo(Gly-Pro) may be derived from the strains, cultures, lysates, extracts, or fermented products thereof.

The "Cyclo(Gly-Pro)" may be also referred to as hexahydropyrrolo[1,2-a]pyrazine-1,4-dione, 3,6-dioxohexahydropyrrolo[1,2-a]pyrazine), glycyl-L-proline lactam, cycloglycyl-L-proline, cyclo-L-prolylglycine, cyclo-GP, or cGP.

In an embodiment, it was confirmed that the mixture of three Streptococcus spp. strains included the Cyclo(Gly-Pro) as an effective substance. In addition, it was confirmed that the Cyclo(Gly-Pro) decreased the expression of CCL17, CCL22, IL-13, IL-22, sIL-2R, and IL-31, and increased the expression of FLG.

Accordingly, another aspect provides a cosmetic composition for prevention or alleviation of skin disease, the cosmetic composition including the Cyclo(Gly-Pro) as an active ingredient.

The Cyclo(Gly-Pro) may be derived from a Streptococcus sp. strain. In an embodiment, the Cyclo(Gly-Pro) may be derived from the three Streptococcus spp. strains, cultures, lysates, extracts, or fermentation products thereof.

The cosmetic composition may additionally include another Streptococcus sp. strain, a culture, a lysate, an extract, or a fermented product thereof, having a skin disease-alleviating effect, thereby exhibiting a synergistic effect.

In the present specification, the term "culture" may be used interchangeably with "culture supernatant", "conditioned culture", or "conditioned medium", and may refer to the entire medium containing a Streptococcus sp. strain or metabolites, extra nutrients, etc. thereof, obtained by culturing the strain for a certain period of time in a medium capable of providing nutrients to enable the strain to grow and survive in vitro. In addition, the culture may refer to a culture from which bacterial cells have been removed from a culture of bacterial cells obtained by culturing the strain.

Meanwhile, the liquid from which the bacterial cells have been removed from the culture is also called "supernatant", may be obtained by allowing the culture to stand still for a certain period of time and collecting only the liquid in the upper layer, excluding the portion that has settled to in the lower layer, or by removing the bacterial cells through filtration, or by centrifuging the culture to remove the precipitates at the bottom and collecting only the top liquid.

The term "bacterial cell" refers to the strain of the present disclosure itself, and may include a strain itself isolated and selected from a skin sample or the like, or a strain isolated from a culture by culturing the strain. The bacterial cell may be obtained by centrifuging the culture and collecting the portion that has precipitated to the lower layer, or the bacterial cell precipitates to the lower layer of the culture by gravity and can therefore be obtained by leaving the culture still for a certain period of time and removing the liquid from the top.

A culture medium and culture conditions for culturing the strains may be appropriately selected or modified by those skilled in the art. For example, the culture may be obtained by culturing the strain in an appropriate medium (e.g., an R2A medium, a TSB medium, or a TSA medium) at an appropriate temperature (e.g., any temperature above 10 °C or below 40 °C) for a certain period of time (e.g., 4 to 168 hours).

The culture may contain a culture itself obtained by culturing the strain, a concentrate thereof, or a lyophilized product thereof. The culture may contain a culture supernatant obtained by removing the strain from the culture, a concentrate thereof, or a lyophilized product thereof.

In an embodiment, the culture supernatant of the strain may be obtained by a process of removing the strain from the strain culture. The process of removing the strain from the strain culture may be performed by centrifuging or filtering the strain culture, but is not limited thereto.

In one or more embodiments, the concentrate may be obtained by a process of concentrating the strain culture itself or the culture supernatant of the strain.

In the present specification, the term "culture filtrate" refers to the liquid remaining after the bacterial cells are filtered out from the culture by centrifugation and filtration. The culture filtrate may contain substances formed and released during the growth process of microorganisms.

In the present specification, the term "lysate" may be used interchangeably with "lysed product", and may refer to a product obtained by disrupting the cell wall of the strain by chemical or physical force. The lysate may contain the lysate itself, a concentrate thereof, or a lyophilized product thereof.

In the present specification, the term "extract" may refer to a substance extracted from the strain itself or from a culture of the strain.

In the present specification, the term "extract of the culture" may refer to an extract from the culture or a concentrate thereof, and may include an extracted solution, a diluted solution or concentrated solution of the extracted solution, a dried product obtained by drying the extracted solution, or a crude product or purified product thereof, or a fraction obtained therefrom.

In the present specification, the term "fermentation product" is used interchangeably with "fermented solution", and may refer to a product produced by decomposition of organic materials by the strain.

The strain, the culture, the lysate, the extract, or the fermentation product thereof may be biologically pure. The strain may be obtained by pure culture.

In the present specification, the term "skin moisturizing" may refer to any action that maintains skin moisture or prevents water loss.

In the present specification, the term "skin barrier strengthening" may refer to any action that, at the outermost part of the skin, enhances the function of the skin barrier that prevents water loss and nutrient loss.

The skin disease may be disease caused by impaired skin barrier function or skin aging, skin wounds, skin scars, or skin inflammation, but are not limited thereto.

The impaired skin barrier function may refer to any change in the skin that occurs as a result of a decreased or impaired function of the skin barrier. For example, the skin disease may include increased skin wrinkles, dryness, dermatitis, atopic dermatitis, allergic dermatitis, acne, or the like.

The skin disease may be at least one selected from the group consisting of atopic dermatitis, contact dermatitis, seborrhea dermatitis, acne vulgaris, and pruritus, but is not limited thereto.

The term "prevention" as used herein may include inhibiting the occurrence of a disease.

The term "alleviation" as used herein may refer to parameters associated with relief or treatment of a condition, and for example, may refer to any action that at least reduces severity of a symptom.

The term "treatment" as used herein may include inhibition, reduction, or elimination of the development of a disease.

The term "including as an active ingredient" as used herein refers that the mixture or Cyclo(Gly-Pro) described in the present specification is added to the extent that can exhibit the aforementioned effects, and that various ingredients are added as secondary ingredients and formulated in various forms for drug delivery and stabilization.

The mixture is included in an amount of 0.0001 to 80 wt%, 0.01 to 70 wt%, 0.01 to 60 wt%, 0.01 to 50 wt%, 0.01 to 40 wt%, 0.01 to 30 wt%, 0.1 to 70 wt%, 0.1 to 60 wt%, 0.1 to 50 wt%, 0.1 to 40 wt%, 0.1 to 30 wt%, 0.5 to 70 wt%, 0.5 to 60 wt%, 0.5 to 50 wt%, 0.5 to 40 wt%, 0.5 to 30 wt%, 1 to 70 wt%, 1 to 60 wt%, 1 to 50 wt%, 1 to 40 wt%, or 1 to 30%, or for example, 30 wt%, based on the total weight of the cosmetic composition, but is not limited thereto.

The Cyclo(Gly-Pro) may be included in an amount of 0.0001 to 10 wt%, 0.01 to 10 wt%, or 0.1 to 10 wt%, based on the total weight of the cosmetic composition, but is not limited thereto.

The mixture may be in a liquid state or a dry state. In an embodiment, the mixture may be in the form of dry powder.

A drying method for preparing the mixture in a dry state may use methods generally used in the art, and is not particularly limited. Non-limiting examples of the drying method are an air drying method, a natural dry method, a spray drying method, a freeze drying method, and the like. These methods may be used alone or at least two methods may be used together.

The cosmetic composition is not particularly limited to a specific formulation, and the formulation may be appropriately selected depending on the intended purpose. The cosmetic composition may have, for example, a solubilized formulation, an emulsified formulation, or a dispersed formulation. The cosmetic composition may be formulated into a softening toner, a nourishing toner, a massage cream, a nourishing cream, an essence, a pack, a gel, an ampoule, or adhesive type cosmetics.

The cosmetic composition may additionally include ingredients commonly used in cosmetic compositions in addition to the effective ingredients disclosed herein, and may include, for example, conventional auxiliary agents and carriers such as antioxidants, stabilizers, solubilizers, surfactants, dispersants, emulsifiers, preservatives, vitamins, pigments, and fragrances.

Another aspect provides a pharmaceutical composition for prevention or alleviation of skin disease, the pharmaceutical composition including three Streptococcus spp. strains, cultures, lysates, extracts, or fermentation products thereof.

Specifically, one aspect provides a pharmaceutical composition for prevention or treatment of skin disease, the pharmaceutical composition including a mixture of (1), (2), and (3) below:
(1) a *Streptococcus pneumoniae* strain, a culture, a lysate, an extract, or a fermentation product thereof;
(2) a *Streptococcus infantis* strain, a culture, a lysate, an extract, or a fermentation product thereof; and
(3) a *Streptococcus mitis* strain, a culture, a lysate, an extract, or a fermentation product thereof.

Another aspect provides a pharmaceutical composition for prevention or treatment of skin disease, the pharmaceutical composition including Cyclo(Gly-Pro) as an active ingredient.

The pharmaceutical composition may include the mixture or the Cyclo(Gly-Pro) in a pharmaceutically effective amount.

The pharmaceutical composition may further include a pharmaceutically acceptable carrier.

The term "pharmaceutically effective amount" as used herein refers to an amount sufficient to achieve the efficacy or activity of the active ingredient.

The pharmaceutical composition may further include a diluent or a carrier. The diluent may be lactose, corn starch, soybean oil, microcrystalline cellulose, mannitol, or a combination thereof. The carrier may include an excipient, a disintegrant, a binding agent, a glydent, or a combination thereof. The excipient may include microcrystalline cellulose, lactose, low-substituted hydroxy cellulose, or a combination thereof. The disintegrant may include carboxymethyl cellulose calcium, sodium starch glycolate, anhydrous calcium hydrogen phosphate, or a combination thereof. The binder may include polyvinylpyrrolidone, low-substituted hydroxypropyl cellulose, hydroxypropyl cellulose, or a combination thereof. The glydent may include magnesium stearate, silicon dioxide, talc, or a combination thereof.

The pharmaceutical composition may be formulated for oral or parenteral administration. The formulation for oral administration may include granules, powders, liquids, tablets, capsules, dry syrups, or a combination thereof. The formulation for parenteral administration may include injections, ointments, or the like.

Alternatively, the composition may be a composition for external use on skin.

Such preparations for external use on skin may include creams, gels, ointments, skin emulsifiers, skin suspensions, transdermal delivery patches, drug-containing bandages, lotions, or a combination thereof.

Regarding the preparations for external use on skin in the present disclosure, ingredients commonly used in the preparations for external use on skin, such as cosmetics or medicines, for example, aqueous ingredients, oily ingredients, powder ingredients, alcohols, moisturizers, thickeners, ultraviolet absorbers, whitening agents, preservatives, antioxidants, surfactants, fragrances, colorants, various skin nutrients, metal ion sequestrants, sugars, or a combination thereof may be appropriately blended as needed.

Another aspect provides a method of preventing, alleviating or treating skin disease, the method including administering an effective amount of the composition to a subject in need thereof.

The term "effective amount" refers to an amount that is effective enough to produce the aforementioned effects.

The subject may be a mammal, for example, a human, a cow, a horse, a pig, a dog, a sheep, a goat, or a cat. The subject may be an entity in need of prevention, alleviation, or treatment of skin disease.

The term "administering" as used herein may be used interchangeably with "introducing" and "grafting", and may be construed as arranging the composition according to an embodiment into the subject by a method or route that results in at least partial localization of the composition according to an embodiment to a desired site.

The administration may be performed by any method known in the art. The administration method including route of administration and the number of doses may be appropriately selected by those skilled in the art. The administration may be performed directly to the subject by any means, for example, by intravenous, intramuscular, oral, transdermal, mucosal, intrabuccal, intranasal, intratracheal, or subcutaneous routes. The administration may be administered systemically or locally. The administration may include application to the skin.

In the present disclosure, the composition according to an embodiment may be administered into the subject at a daily dose in a range of about 0.1 mg to about 1,000 mg, for example, about 0.1 mg to about 500 mg, about 0.1 mg to about 100 mg, about 0.1 mg to about 50 mg, about 0.1 mg to about 25 mg, about 1 mg to about 1,000 mg, about 1 mg to about 500 mg, about 1 mg to about 100 mg, about 1 mg to about 50 mg, about 1 mg to about 25 mg, about 5 mg to about 1,000 mg, about 5 mg to about 500 mg, about 5 mg to about 100 mg, about 5 mg to about 50 mg, about 5 mg to about 25 mg, about 10 mg to about 1,000 mg, about 10 mg to about 500 mg, about 10 mg to about 100 mg, about 10 mg to about 50 mg, or about 10 mg to about 25 mg. However, the dosage may be variously prescribed depending on factors, such as a formulation method, an administration method, age, weight, gender, and medical conditions of a patient, food, administration time, an administration route, an excretion rate, and response sensitivity, and a person skilled in the art may appropriate adjust the dosage in consideration of these factors. The number of administration may be once a day or at least twice a day within the range of clinically acceptable side effects, and the administration may be performed at a single site or at least two sites, daily or every 2 days to 5 days. The total number of administration days may be 1 day to 30 days per treatment. As needed, the same treatment may be repeated after the a suitable period of time. For animals other than humans, the same dosage per kg as for humans may be used, or for example, a dosage converted from the aforementioned dosage by the volume ratio (e.g., average value) of the organ (e.g., heart, etc.) between a target animal and the human may be used.

Another aspect provides use of the three Streptococcus spp. strains, cultures, lysates, extracts, or fermentation products thereof, or Cyclo(Gly-Pro), in the manufacture of a composition for prevention, alleviation, or treatment of skin disease.

Another aspect provides use of the three Streptococcus spp. strains, cultures, lysates, extracts, or fermentation products thereof, or Cyclo(Gly-Pro), in the manufacture of a therapeutic medicament for prevention, alleviation, or treatment of skin disease.

Redundancies are omitted in consideration of the complexity of the present specification, and terms not otherwise defined herein may have the meanings commonly used in the technical field to which the present invention pertains.

### Advantageous Effects of Invention

A composition according to an aspect includes three Streptococcus spp. strains, cultures, lysates, extracts, or fermentation products thereof, or a core efficacy substance thereof, Cyclo(Gly-Pro), thereby exhibiting excellent effects in the prevention, alleviation, or treatment of skin disease. Therefore, the composition may be used as a composition for the prevention, alleviation, or treatment of skin disease.

### Brief Description of the Drawings

FIG. 1 is a graph showing relative expression levels of a thymic stromal lymphopoietin (TSLP) gene in response to treatment with three culture broth filtrates of a Streptococcus strain individually and in combination, in an inflammatory skin cell model.
FIG. 2A is a graph showing changes in IGA scores according to use of a product including a mixture according to an embodiment and a control product.
FIG. 2B is a graph showing changes in EASI scores according to use of a product including a mixture according to an embodiment and a control product.
FIG. 3A is a graph showing changes in skin moisture content according to use of a product including a mixture according to an embodiment and a control product.
FIG. 3B is a graph showing changes in transdermal water loss content according to use of a product including a mixture according to an embodiment and a control product.
FIG. 4A is a graph showing changes in response rates for mild or worse itchiness according to use of a product including a mixture according to an embodiment and a control product.
FIG. 4B is a graph showing changes in response rates for severe or worse sleep disturbance according to use of a product including a mixture according to an embodiment and a control product.
FIG. 5A is a graph showing differences in relative abundance of microorganisms of the genus *Streptococcus* before and after use of a product including a mixture according to an embodiment.
FIG. 5B is a graph showing differences in relative abundance of *Staphylococcus aureus* before and after use of a product including a mixture according to an embodiment.
FIG. 6 is a graph showing mass spectra of a Cyclo(Gly-Pro) standard material, a Strain CX Solution, and three culture broth filtrates of a *Streptococcus* strain.
FIG. 7A is a GC-FID chromatogram of a mixed solution of a Cyclo(Gly-Pro) standard (STD) substance and an internal standard (ISTAD) substance.
FIG. 7B is a GC-FID chromatogram of samples in which a Strain CX Solution standard material is added at different concentrations.
FIG. 7C is a graph showing a calibration curve of Cyclo(Gly-Pro) calculated by a standard addition method.
FIGS. 8A to 8F are graphs showing changes in relative expression levels of inflammatory marker genes related to atopic dermatitis according to Cyclo(Gly-Pro) treatment in an inflammatory skin cell model mimicking atopic dermatitis. A: CCL17, B: CCL22, C: IL-13, D: IL-22, E: sIL-2R, F: IL-31.
FIG. 9 is a graph showing changes in relative expression levels of filaggrin genes according to Cyclo(Gly-Pro) treatment in an inflammatory skin cell model mimicking atopic dermatitis.

### Best Mode for Carrying out the Invention

Hereinafter, the present disclosure will be described in detail with reference to Examples to explain the present invention in detail. However, the Examples according to the present disclosure may be modified in various different forms, and the scope of the present disclosure should not be construed as being limited to the Examples described below. The Examples of the present disclosure are provided to more fully explain the present disclosure to those having average knowledge in the art.

### Example 1. Preparation of control product and test products containing mixture of strain culture broth filtrates

An *S. pneumoniae* CX-2 strain (accession No: KCCM12005P), an *S. infantis* CX-4 strain (accession No.: KCCM12642P), and an *S. mitis* CX-8 strain (accession No.: KCCM12656P) were each inoculated into a tryptic soy broth (TSB) medium and cultured for 30 to 72 hours. Each culture was centrifuged at 5,000 g for 15 minutes, and the supernatant was filtered through a filter having a 0.45-µm pore size. The culture broth filtrates of the three obtained strains were mixed at a weight ratio of 1:1:1 to prepare a Streptococcus postbiotic mixture (named "Strain CX Solution").

To evaluate the efficacy of the Strain CX Solution in alleviating atopic dermatitis, a test product to be applied to clinical trials was manufactured. Here, a minimal skeletal essence formulation that does not affect the efficacy evaluation of the Strain CX Solution was applied. A product that does not contain the Strain CX Solution was set as a control group, and a test product containing 1 wt% Strain CX Solution was named 'Strain CX Essence (low concentration)' and a test product containing 30 wt% Strain CX Solution was named 'Strain CX Essence (high concentration)'.

Specific ingredient compositions of the control group, the Strain CX Essence (low concentration)-treated group, and the Strain CX Essence (high concentration)-treated group are shown in Table 1. The 16S rRNA sequences (complementary DNA) of the S. *pneumoniae* strain CX-2, the *S. infantis* strain CX-4, and the S. *mitis* CX-8 strain are shown in Table 2.

**[Table 1]**

| **Composi tion** | **Ingredient name** | **Control group** | **Strain CX essence (low concentration )** | **Strain CX essence (high concentration )** |
|---|---|---|---|---|
| **Solvent** | **Purified water** | 77.79 | 76.79 | 47.79 |
| **Preserva tive** | **1,2-hexanediol** | 2 | 2 | 2 |
| **Neutraliz er (buffer)** | **Tromethamine** | 0.16 | 0.16 | 0.16 |
| | (Product name: Tris amino ultra pc) | | | |
| **Preserva tive** | **Ethylhexyl glycerin** | 0.05 | 0.05 | 0.05 |
| | (Product name: Sensiva sc 50) | | | |
| **Thickene r** | **Carbomer** | 20 | 20 | 20 |
| | (Product name: Carbopol 980 (1% (w/w) aqueous solution)) | | | |
| **Efficacy ingredie nt** | **Strain CX Solution** | 0 | 1 | 30 |
| **Total (wt%)** | | 100 | 100 | 100 |

**[Table 2]**

| **SEQ ID NO:** | **Name** | **Sequence (5'→3')** |
|---|---|---|
| 1 | Streptoco ccus pneumoni ae CX-2 | gagttgcgaacgggtgagtaacgcgtaggtaacctgcctggtagcgggggataactattgga aacgatagctaataccgcataagagtagatgttgcatgacatttgcttaaaaggtgcacttgca tcactaccagatggacctgcgttgtattagctagttggtggggtaacggctcaccaaggcgac gatacatagccgacctgagagggtgatcggccacactgggactgagacacggcccagact cctacgggaggcagcagtagggaatcttcggcaatggacggaagtctgaccgagcaacgc cgcgtgagtgaagaaggttttcggatcgtaaagctctgttgtaagagaagaacgagtgtgag agtggaaagttcacactgtgacggtatcttaccagaaagggacggctaactacgtgccagc agccgcggtaatacgtaggtcccgagcgttgtccggatttattgggcgtaaagcgagcgcag gcggttagataagtctgaagttaaaggctgtggcttaaccatagtaggctttggaaactgtttaa cttgagtgcaagaggggagagtggaattccatgtgtagcggtgaaatgcgtagatatatgga ggaacaccggtggcgaaagcggctctctggcttgtaactgacgctgaggctcgaaagcgtg gggagcaaacaggattagataccctggtagtccacgctgtaaacgatgagtgctaggtgtta gaccctttccggggtttagtgccgtagctaacgcattaagcactccgcctggggagtacgacc gcaaggttgaaactcaaaggaattgacgggggcccgcacaagcggtggagcatgtggttta attcgaagcaacgcgaagaaccttaccaggtcttgacatccctctgaccgctctagagatag agttttccttcgggacagaggtgacaggtggtgcatggttgtcgtcagctcgtgtcgtgagatgtt gggttaagtcccgcaacgagcgcaacccctattgttagttgccatcatttagttgggcactctag cgagactgccggtaataaaccggaggaaggtggggatgacgtcaaatcatcatgcccctta tgacctgggctacacacgtgctacaatggctggtacaacgagtcgcaagccggtgacggca agctaatctcttaaagccagtctcagttcggattgtaggctgcaactcgcctacatgaagtcgg aatcatccgatcc |
| 2 | Streptoco ccus infantis CX-4 | gacgaacgctggcggcgtgcctaatacatgcaagtagaacgctgaaggaggagcttgctct tctggatgagttgcgaacgggtgagtaacgcgtaggtaacctgcctggtagcgggggataac tattggaaacgatagctaataccgcataacagtagatatcgcatgatagctgcttgaaaggtg caattgcaccactaccagatggacctgcgttgtattagctagttggtgaggtaacggctcacca aggcaacgatacatagccgacctgagagggtgatcggccacactgggactgagacacgg cccagactcctacgggaggcagcagtagggaatcttcggcaatggacggaagtctgaccg agcaacgccgcgtgagtgaagaaggttttcggatcgtaaagctctgttgtaagagaagaacg agtgtgagagtggaaagttcacactgtgacggtatcttaccagaaagggacggctaactacg tgccagcagccgcggtaatacgtaggtcccgagcgttgtccggatttattgggcgtaaagcga gcgcaggcggttagataagtctgaagttaaaggctgtggcttaaccatagtacgctttggaaa ctgtttaacttgagtgcaagaggggagagtggaattccatgtgtagcggtgaaatgcgtagat atatggaggaacaccggtggcgaaagcggctctctggcttgtaactgacgctgaggctcga aagcgtggggagcaaacaggattagataccctggtagtccacgccgtaaacgatgagtgct aggtgttagaccctttccggggtttagtgccgaagctaacgcattaagcactccgcctgggga gtacgaccgcaaggttgaaactcaaaggaattgacgggggcccgcacaagcggtggagc atgtggtttaattcgaagcaacgcgaagaaccttaccaggtcttgacatccctctgaccgctct agagatagagttttccttcgggacagaggtgacaggtggtgcatggttgtcgtcagctcgtgtc gtgagatgttgggttaagtcccgcaacgagcgcaacccctattgttagttgccatcatttagttg ggcactctagcgagactgccggtaataaaccggaggaaggtggggatgacgtcaaatcat catgccccttatgacctgggctacacacgtgctacaatggttggtacaacgagtcgcaagcc ggtgacggcaagctaatctcttaaagccaatctcagttcggattgtaggctgcaactcgcctac atgaagtcggaatcgctagtaatcgcggatcagcacgccgcggtgaatacgttcccgggcct tgtacacaccgcccgtcacaccacgagagtttgtaacacccgaagtcggtgaggtaaccatt tggagccagccgcctaaggtgggatagatgattggggtg |
| 3 | Streptoco ccus mitis CX-8 | ttttgagtttgatcctggctcaggacgaacgctggcggcgtgcctaatacatgcaagtagaac gctgaaggaggagcttgcttctccggatgagttgcgaacgggtgagtaacgcgtaggtaacc tgcctggtagcgggggataactattggaaacgatagctaataccgcataagagtagatgttgc atgacatttgcttaaaaggtgcaattgcatcactaccagatggacctgcgttgtattagctagttg gtggggtaacggctcaccaaggcgacgatacatagccgacctgagagggtgatcggccac actgggactgagacacggcccagactcctacgggaggcagcagtagggaatcttcggcaa tggacggaagtctgaccgagcaacgccgcgtgagtgaagaaggttttcggatcgtaaagct ctgttgtaagagaagaacgagtgtgagagtggaaagttcacactgtgacggtatcttaccag aaagggacggctaactacgtgccagcagccgcggtaatacgtaggtcccgagcgttgtccg gatttattgggcgtaaagcgagcgcaggcggttagataagtctgaagttaaaggctgtggctt aaccatagtacgctttggaaactgtttaacttgagtgcaagaggggagagtggaattccatgt gtagcggtgaaatgcgtagatatatggaggaacaccggtggcgaaagcggctctctggcttg taactgacgctgaggctcgaaagcgtggggagcaaacaggattagataccctggtagtcca cgccgtaaacgatgagtgctaggtgttagaccctttccggggtttagtgccgcagctaacgcat taagcactccgcctggggagtacgaccgcaaggttgaaactcaaaggaattgacgggggc ccgcacaagcggtggagcatgtggtttaattcgaagcaacgcgaagaaccttaccaggtctt gacatccctctgaccgctctagagatagagttttccttcgggacagaggtgacaggtggtgcat ggttgtcgtcagctcgtgtcgtgagatgttgggttaagtcccgcaacgagcgcaacccctattgt tagttgccatcatttagttgggcactctagcgagactgccggtaataaaccggaggaaggtgg ggatgacgtcaaatcatcatgccccttatgacctgggctacacacgtgctacaatggctggta caacgagtcgcaagccggtgacggcaagctaatctcttaaagccagtctcagttcggattgta ggctgcaactcgcctacatgaagtcggaatcgctagtaatcgcggatcagcacgccgcggt gaatacgttcccgggccttgtacacaccgcccgtcacaccacgagagtttgtaacacccgaa gtcggtgaggtaaccttttaggagccagccgcctaaggtgggatagatgattggggtgaagtc gtaacaagtcagccgtttgggaa |

### Experimental Example 1. Evaluation of antipruritic efficacy of culture filtrate of microorganism of genus Streptococcus

The culture broth filtrates of the *S. pneumoniae* CX-2 strain, the S. *infantis* CX-4 strain, and the S. *mitis* CX-8 strain were subjected to the evaluation of the antipruritic efficacy.

Specifically, HaCaT cells, a human keratinocyte cell line, were cultured in a Dulbecco's modified Eagle's medium (DMEM) (Gibco 1210-0038) supplemented with 10 % fetal bovine serum, and the culturing was performed at 37 °C in a 5 % CO₂ incubator. The medium was changed every 3 to 4 days, and the cells were subcultured when they proliferated excessively. Afterwards, the cells were seeded at 5 x 10⁵ cells/well, and washed with phosphate-buffered saline solution (PBS) after 24 hours of the culturing. The resulting cells were seeded into each well containing an FBS-free DMEM, and 10 µg/mL of Poly I:C and 10 ng/mL of recombinant human (rh) IL-4 were added thereto to induce inflammation in the keratinocyte cell line. Next, *S. pneumoniae* CX-2 culture broth filtrate (1% (w/w)), *S. infantis* CX-4 culture broth filtrate (1% (w/w)), *S. mitis* CX-8 culture broth filtrate (1% (w/w)), a mixed solution (1% (w/w)) containing *S. pneumoniae* CX-2 culture broth filtrate, and *S. mitis* CX-8 culture broth filtrate at 1:1, and a mixed solution (1% (w/w)) containing *S. pneumoniae* CX-2 culture broth filtrate, *S. infantis* CX-4 culture broth filtrate, and *S. mitis* CX-8 culture broth filtrate at 1:1:1 were each treated and additionally cultured for 4 hours. Here, 1 µm dexamethasone was used as a positive control.

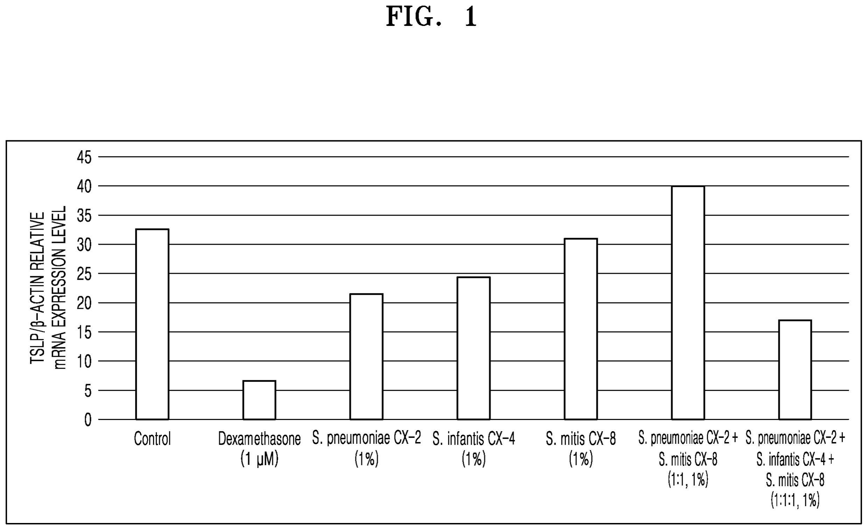

Afterwards, RNA was isolated from the cells of each sample using triazole (RNA iso, DAKARA, Japan), and quantified at 260 nm using a nanodrop. Then, cDNA was synthesized in an amplifier (C1000 Thermal Cycler, Bio-Rad, USA) using 2 µg of RNA of each sample. CyberGreen (SYBR Green supermix, Applied Biosystems, USA) and primers for target genes (primers for thymic stromal lymphopoietin (TSLP)) were added to the synthesized cDNA, and real-time PCR reaction was performed thereon on a real-time PCR machine (Step One Plus, Applied Biosystems, USA). The primer sequences for TSLP used were as follows: 5-TCGGAAACTCAGATAAATGCTAC-3 (forward primer; SEQ ID NO: 4), 5-TGAAGCGACGCCACAATC-3 (reverse primer; SEQ ID NO: 5). The expression levels of the genes were finally analyzed through correction for the β-actin gene, and the results are shown in FIG. 1.

FIG. 1 is a graph showing the relative expression levels of a TSLP gene in response to treatment with three culture broth filtrates of a Streptococcus strain individually and in combination, in an inflammatory skin cell model.

As a result, as shown in FIG. 1, it was confirmed that, compared to the untreated control group, the three culture broth filtrates of microorganisms of the genus Streptococcus reduced the expression of TSLP which is a factor related to itch. The mixed solution (1% (w/w)) containing each culture broth filtrates at 1:1:1 showed a synergistic effect, resulting in improved efficacy over each culture broth filtrate treated individually. In addition, the mixed solution containing the *S. pneumoniae* CX-2 culture filtrate and the *S*. *mitis* CX-8 culture filtrate at 1:1 did not show any synergistic effect, suggesting that the *S*. *infantis* CX-4 culture broth filtrate played a key role in the synergistic effect.

### Experimental Example 2. Evaluation of efficacy of alleviating atopic dermatitis

The two test products and the control product of Example 1 were subjected to a clinical efficacy evaluation for alleviating atopic dermatitis. The evaluation was commissioned by the Korean Skin Research Center (KSRC).

The study was conducted in 98 subjects aged 13 to 70 years (mean 42.77±13.92 years) diagnosed with atopic dermatitis (IGA score 3 or lower). An independent statistician randomly assigned patients to the two test product groups or the control group at a ratio of 1:1:1 according to a stratified block randomization method using SAS version 9.3 (SAS Institute, Cary, NC). A blinded kit including the two test products or the control product was used, and at randomization, all subjects were instructed to regularly use a moisturizer regularly for 8 weeks on their skin with atopic dermatitis and all types of dry skin. The subjects applied the test product twice daily (morning and evening) to the test area (lesion area) for 8 weeks, with clinical evaluations at weeks 0, 4, and 8. The results of the analysis were verified for statistical significance using the SPSS Package Program (IBM, USA), and the statistical significance level was set at a p-value of less than 0.05.

### 2-1. Visual evaluation of atopic dermatitis

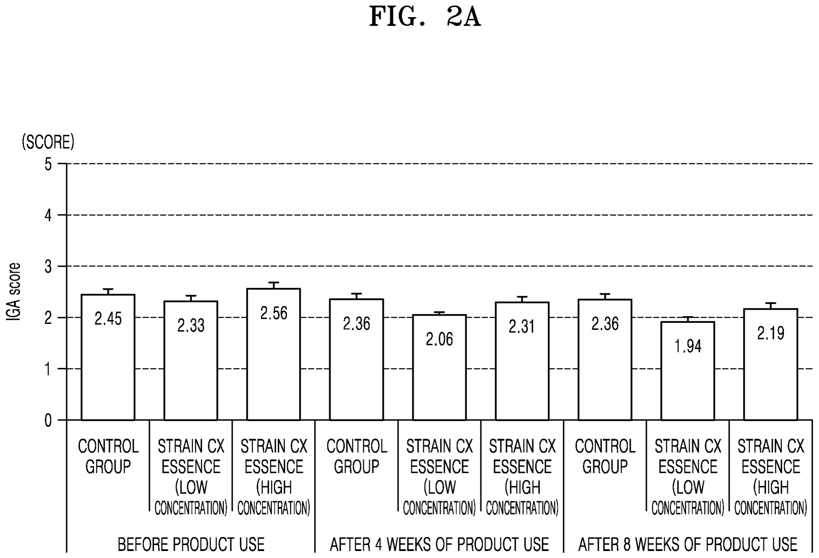

The Investigator's Global Assessment (IGA) score for the test site (lesion site) was measured and shown in FIG. 2A. As shown in FIG. 2A, the Strain CX Essence (low concentration) group and the Strain CX Essence (high concentration) group tended to show a further decrease in the IGA scores after product use compared to the control group. The IGA scores of the Strain CX Essence (low concentration) group and the Strain CX Essence (high concentration) group decreased by 11.69 % and 9.76 %, respectively, after 4 weeks of product use, and decreased by 16.88 % and 14.63 %, respectively, after 8 weeks of product use.

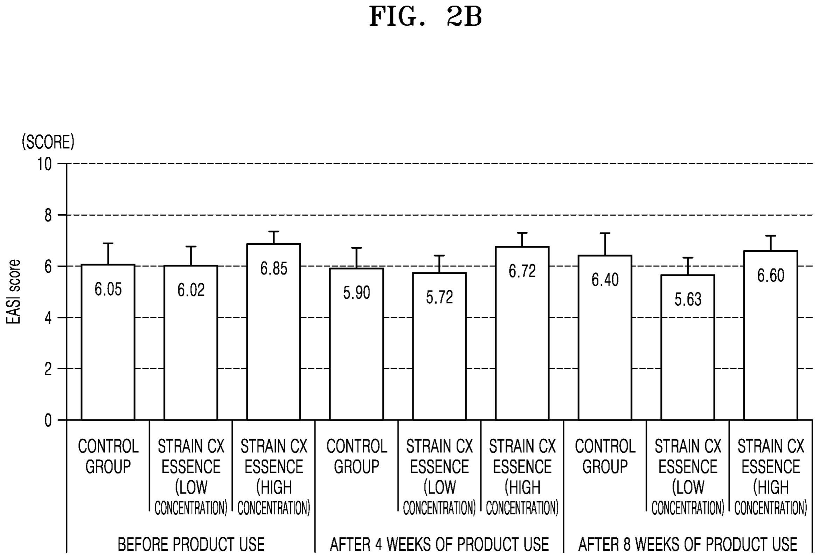

The Eczema Area and Severity Index (EASI) score for the test site (lesion site) was measured and shown in FIG. 2B. As shown in FIG. 2B, the Strain CX Essence (low concentration) group and the Strain CX Essence (high concentration) group tended to show a further decrease in the EASI scores after product use compared to the control group. The EASI score of the Strain CX Essence (low concentration) group decreased by 5.03 % after 4 weeks of product use, and decreased by 6.54 % and 3.70 % in the Strain CX Essence (low concentration) group and the Strain CX Essence (high concentration) group, respectively, after 8 weeks of product use.

### 2-2. Evaluation of skin barrier function

To evaluate the skin barrier function at the test site (lesion site), skin moisture content and transepidermal water loss content were measured. The measurement was conducted in accordance with the 'Guidelines for human application tests of cosmetics that help alleviation of itchiness by restoring the function of the skin barrier' of the Ministry of Food and Drug Safety.

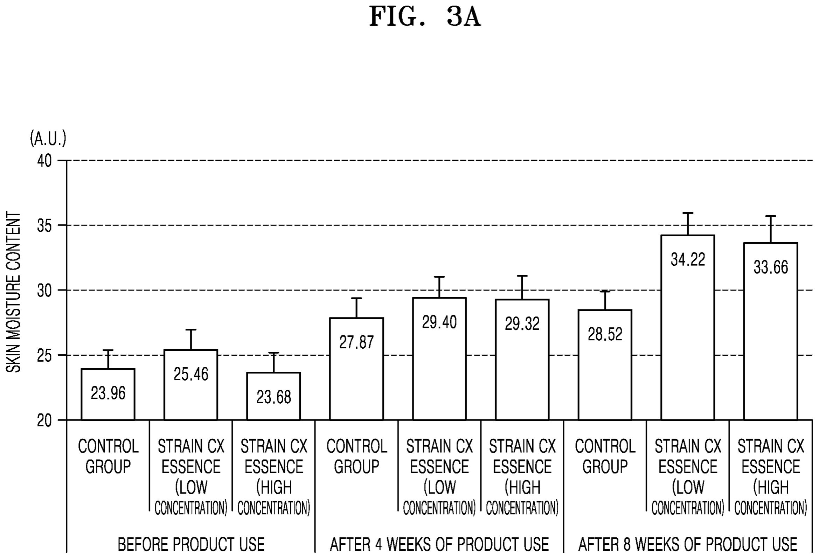

Skin moisture content was analyzed using Corneometer^{®}, and the results are shown in FIG. 3A. As shown in FIG. 3A, both the Strain CX Essence (low concentration) group and the Strain CX Essence (high concentration) group showed a tendency to increase the skin moisture content. The change rate in skin moisture contents of the Strain CX Essence (low concentration) group and the Strain CX Essence (high concentration) group was 15.51 % and 23.82 %, respectively, after 4 weeks of product use, and 34.43 % and 42.14 %, respectively, after 8 weeks of product use.

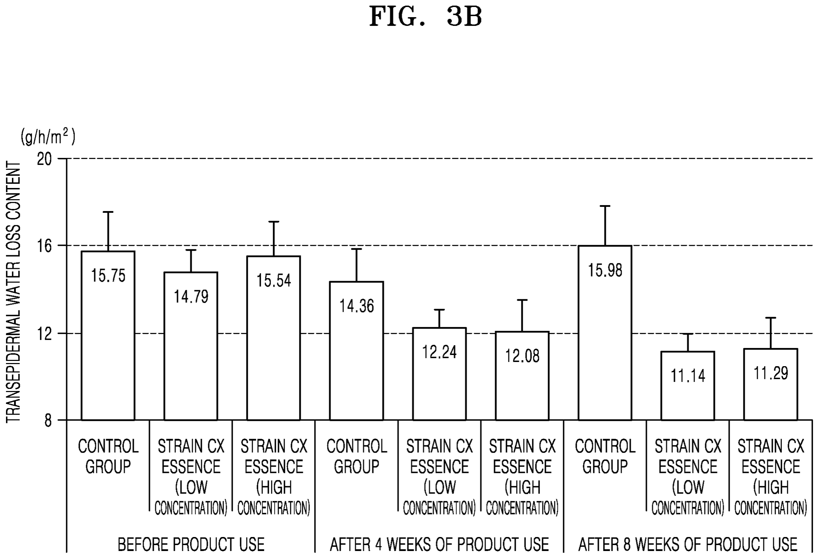

Transepidermal water loss content was analyzed using Tewameter^{®}, and the results are shown in FIG. 3B. As shown in FIG. 3B, both the Strain CX Essence (low concentration) group and the Strain CX Essence (high concentration) group showed a tendency to decrease the transepidermal water loss content. The change rate in transepidermal water loss contents of the Strain CX Essence (low concentration) group and the Strain CX Essence (high concentration) group was decreased by 3.95 %, 5.64 %, respectively, after 4 weeks of product use, and decreased by 8.76 % and 9.98 %, respectively, after 8 weeks of product use.

### 2-3. Self-evaluation of skin itchiness

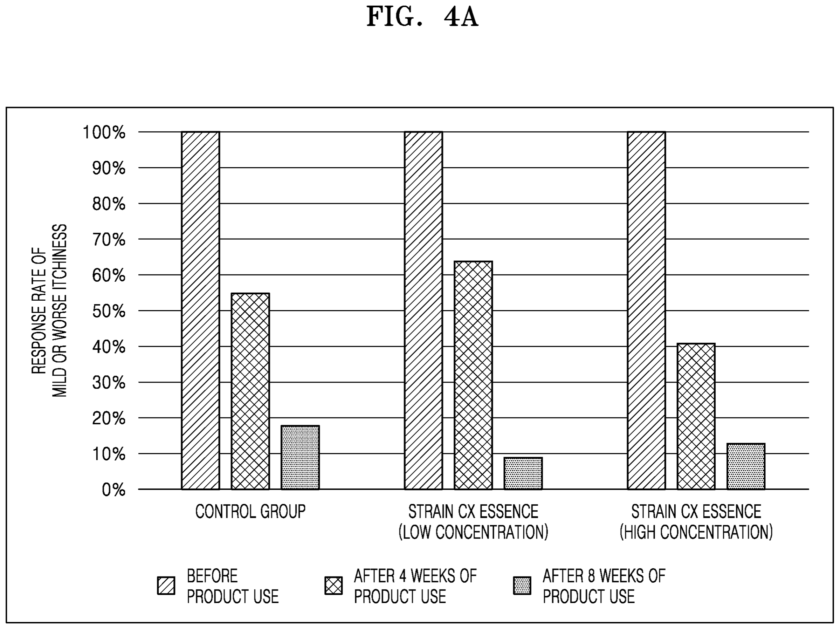

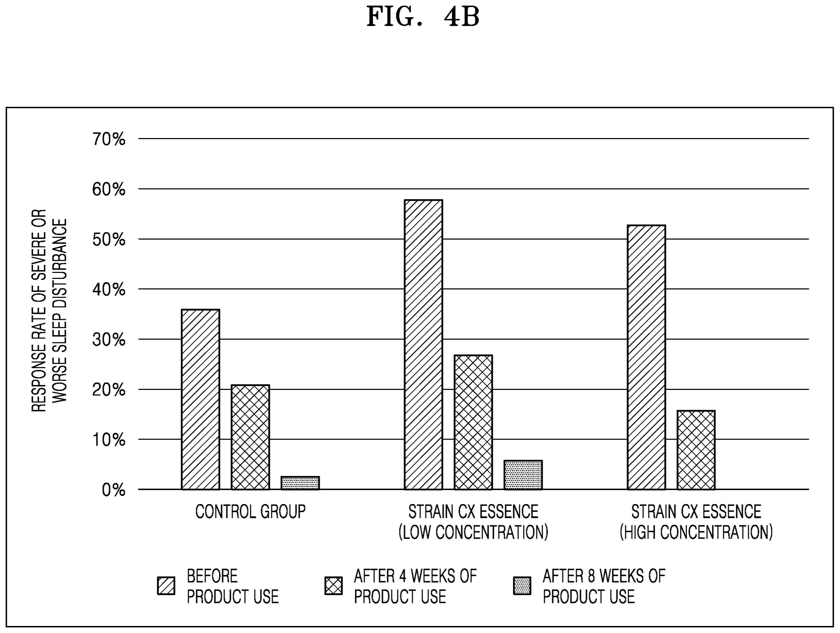

The pruritus visual analog scale (PVAS) was accessed for self-evaluation of itchiness at the test site (lesion site). The assessment was conducted in accordance with the 'Guidelines for human application tests of cosmetics that help alleviation of itchiness by restoring the function of the skin barrier' of the Ministry of Food and Drug Safety. The results are shown in FIGS. 4A and 4B.

As shown in FIG. 4A, the number of subjects who responded that the itching level at the test area (lesion area) was mild or worse decreased from 100 % before product use to 64 % and 41 % after 4 weeks of product use in the Strain CX Essence (low concentration) group and the Strain CX Essence (high concentration) group, respectively, and decreased to 9 % and 13 % after 8 weeks of product use.

In addition, as shown in FIG. 4B, the number of subjects who reported severe or worse sleep disturbance decreased from 58 % and 53 % before product use to 27 % and 16 % after 4 weeks of product use in the Strain CX Essence (low concentration) group and the Strain CX Essence (high concentration) group, respectively, and decreased to 6 % and 0 % after 8 weeks of product use.

### 2-4. Evaluation of skin microbiome improvement

The two test products of Example 1 was evaluated for their efficacy of improving the skin microbiome at lesion sites of patients with atopic dermatitis.

First, metagenomic DNA (mDNA) was extracted from skin swab samples using the QIAamp PowerFecal pro DNA kit (Qiagen, Germany). After confirming the purity and yield of the extracted DNA using Bioanalyzer (Agilent 2100, USA), a 16S V3-V4 metagenome sequencing library was constructed according to the Illumina library construction workflow. PCR amplification for library construction was performed using 2X KAPA HiFi Hot Start Ready Mix (Roche, Germany). General primer pairs specific for the bacterial 16S V3-V4 hyper-variable region used are as follows.

16S 341F forward primer: 5'-
TCGTCGGCAGCGTCAGATGTGTATAAGAGACAGCCTACGGGNGGCWGCAG-3'
(SEQ ID NO: 6)

16S 806R reverse primer: 5'-
GTCTCGTGGGCTCGGAGATGTGTATAAGAGACAGGACTACHVGGGTATCTAATCC-
3' (SEQ ID NO: 7)

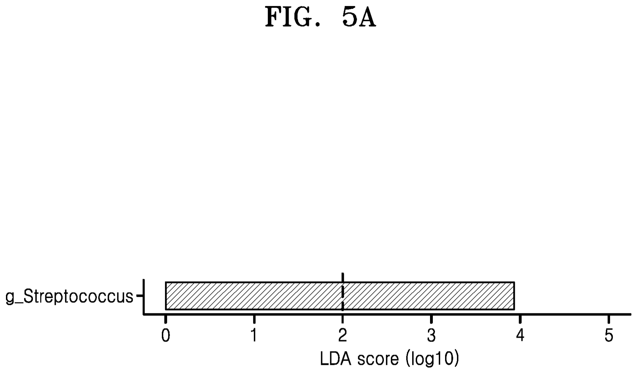

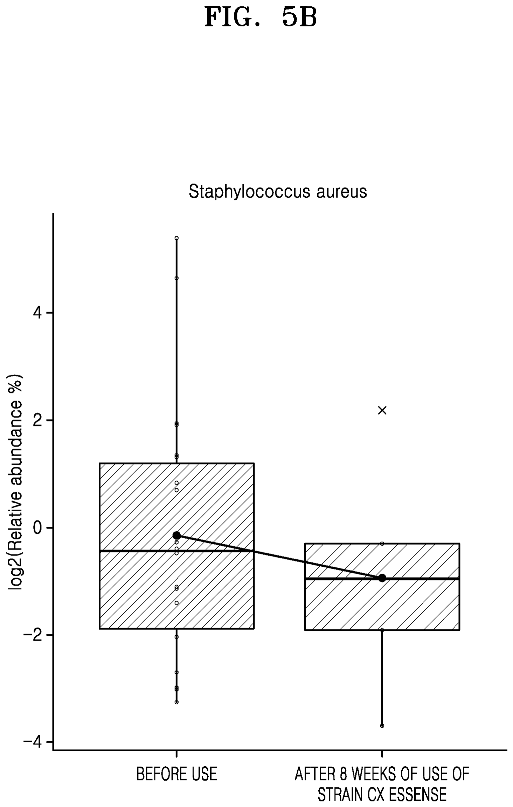

Following the PCR amplification process, the amplicon product was purified using AMPure XP beads (Beckman Coulter, USA). Subsequently, Illumina adapters and multiplex indices (Nextera XD Index kit; Illumina, USA) were attached to the 16S amplicon by additional PCR. The final PCR products were purified again using AMPure XP beads, and 16S V3-V4 metagenome sequencing was performed according to the Illumina Miseq (2 × 300) paired-end sequencing workflow (Illumina, USA). For skin microbiome community analysis, the generated 16S metagenome sequencing read data were applied to the QIIME2TM microbiome bioinformatics analysis pipeline, and Amplicon Sequence Variants (ASVs) clustering was performed using the Divisive Amplicon Denoising Algorithm 2 (DADA2). The relative microbial abundance for each sample was calculated using the Naive Bayes classifier at a confidence threshold of 0.7 %, and taxonomic assignment was performed using the Silva v138 reference database. Afterwards, the taxa *(Streptococcus)* showing significant differences in the relative microbial abundance between each comparison group were identified by linear discriminant analysis effect size (LEfSe) analysis, and the relative abundance of S. *aureus* was compared by the vsearch-sklearn feature classifier method, and the results are shown in FIGS. 5A and 5B.

As shown in FIG. 5A, the relative abundance of microorganisms of the genus Streptococcus increased after 8 weeks of using the Strain CX essence product.

In addition, as shown in FIG. 5B, the relative abundance of S. *aureus* tended to decrease after 8 weeks of using the Strain CX essence product.

### Experimental Example 3. Identification and verification of core efficacy substance in Strain CX Solution

To identify core substances responsible for alleviation of atopic dermatitis improvement efficacy in Strain CX Solution, substance analysis and in vitro efficacy evaluation were conducted.

### 3-1. Qualitative analysis of substances in Strain CX Solution

For material analysis of the Strain CX Solution, GC-MS qualitative analysis was performed.

To prepare samples at the same concentration (100 mg/mL), each of a lyophilisate of the Strain CX Solution (1.12 g), a lyophilisate of the *S. pneumoniae* CX-2 culture broth filtrate (1.25 g), a lyophilisate of the *S*. *infantis* CX-4 culture broth filtrate (1.22 g), and a lyophilisate of the *S*. *mitis* CX-8 culture broth filtrate (1.14 g) was added to 11.2 mL, 12.5 mL, 12.2 mL, and 11.4 mL of a mixed solution of chloroform:MeOH (2:1, v/v), respectively. The samples were sonicated thoroughly and then dehydrated by adding anhydrous magnesium sulfate. The supernatant of the dehydrated solution was filtered through a 0.45-µm PVDF syringe filter and used for GC-MS measurements. A standard solution was prepared at 1 mg/mL in the same solvent. GC-MS (PerkinElmer, Clarus 600/600T, USA) was used as the measuring equipment for qualitative analysis, and DB-5 (Agilent) was used as the column. The mass analysis range was 45 to 600 amu, and the temperatures of the inlet, source, and injector were set to 280 °C, 290 °C, and 280 °C, respectively. Oven conditions were set to start at 40 °C, heat up to 300 °C at a heating rate of 10 °C/min, and hold for 10 minutes. Helium was used as the carrier gas, and the flow rate of the column was set to 1.5 mL/min with a 10:1 split.

As a result of the analysis, the unique metabolites of the Strain CX Solution, excluding the components detected in the medium, were identified as 3-pyrrolidin-2-yl-propionic acid and Cyclo(Gly-Pro) (syn. hexahydropyrrolo[1,2-a]pyrazine-1,4-dione). Among these substances, additional analysis was conducted targeting the Cyclo(Gly-Pro), which is structurally similar to ketoprofen which is an anti-inflammatory drug well known in the art.

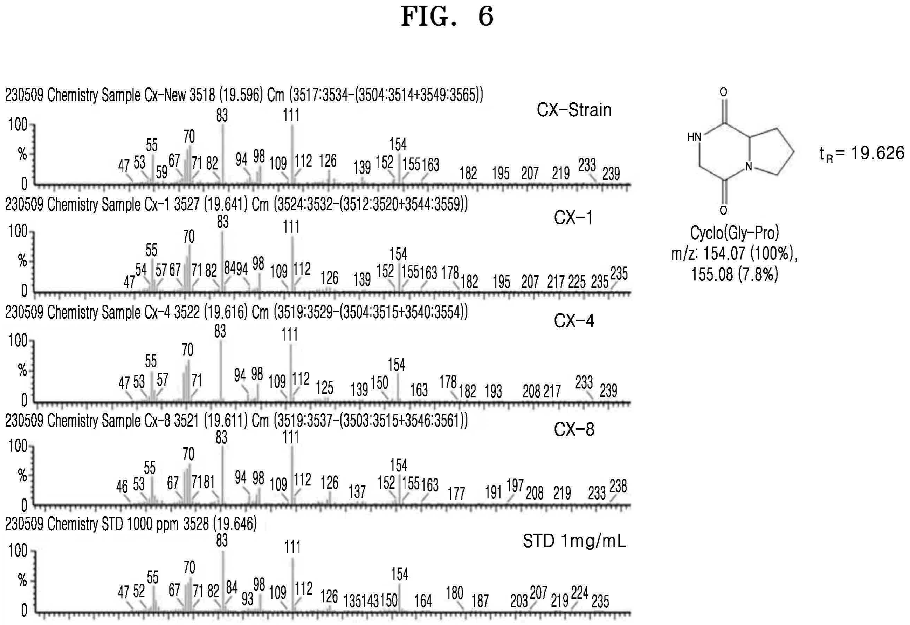

As shown in FIG. 6, the retention time (tR) of the Cyclo(Gly-Pro) standard substance was 19.626 minutes. The same tR was confirmed in each of the Strain CX Solution, the *S. pneumoniae* CX-2 culture broth filtrate, the *S*. *infantis* CX-4 culture broth filtrate, and the *S*. *mitis* CX-8 culture broth filtrate, and the TIC peaks of all samples had the same mass spectra. These results indicate that all samples contain the Cyclo(Gly-Pro).

### 3-2. Quantitative analysis of Cyclo(Gly-Pro) in Strain CX Solution

GC-FID analysis was conducted to quantify Cyclo(Gly-Pro) in the Strain CX Solution.

The standard addition method was used for quantitative analysis, and dibutyl phthalate was added as an internal standard (ISTD) substance to compensate for signal reduction due to interfering substances in the sample. 2.62 g of the lyophilisate of the Strain CX Solution was added to 26.2 mL of a mixed solution of chloroform:MeOH (2:1, v/v) and the concentration was adjusted to 100 mg/mL. The resulting solution was then sonicated thoroughly, and then dehydrated with anhydrous magnesium sulfate added thereto. The dehydrated supernatant was filtered through a 0.45-µm PVDF syringe filter. The standard solution was prepared at 1 mg/mL by adding 33.1 mg of the Cyclo(Gly-Pro) standard substance in the same solvent, and the ISTD substance was prepared at 0.5 mg/mL by adding 10 mg of dibutyl phthalate in the same solvent.

**[Table 3]**

| | A (0 ppm) | B (111 ppm) | C (222 ppm) | D (333 ppm) | E (444 ppm) |
|---|---|---|---|---|---|
| Strain CX Solution (100 mg/mL) | 0.8 | 0.8 | 0.8 | 0.8 | 0.8 |
| Standard solution (1 mg/mL) | 0 | 0.2 | 0.4 | 0.6 | 0.8 |
| Internal standard (ISTD) solution (0.5 mg/mL) | 0.2 | 0.2 | 0.2 | 0.2 | 0.2 |
| Solvent | 0.8 | 0.6 | 0.4 | 0.2 | 0 |

Table 3 shows compositions of the solutions prepared according to the standard addition method. Completed samples from A to E were analyzed using GC-FID (Agilent, 7820A, USA) and HP-5 (Agilent) column. The FID temperature was set to 300 °C, the carrier gas was helium, and the flow rates of the detector were set to 25, 30, and 400 mL/min for nitrogen, hydrogen, and purified air, respectively. The inlet temperature was 280 °C, and the oven conditions were set to start at 40 °C, hold for 4 minutes, heat up to 300 °C at a heating rate of 10 °C/min, and hold for 10 minutes. The flow rate of the column was set to 1.5 mL/min with a 1:1 split.

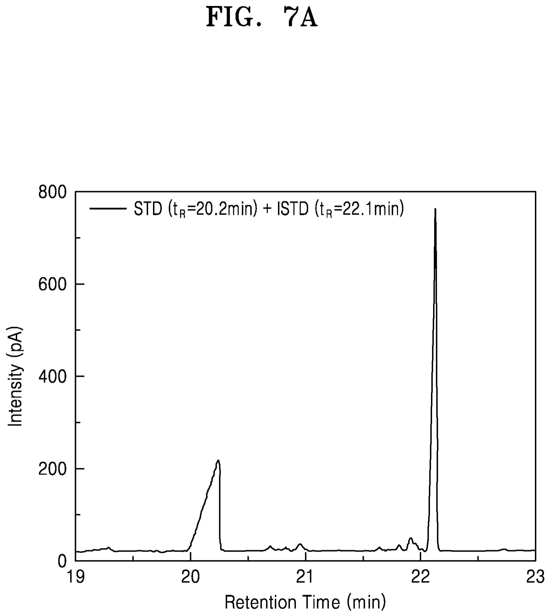

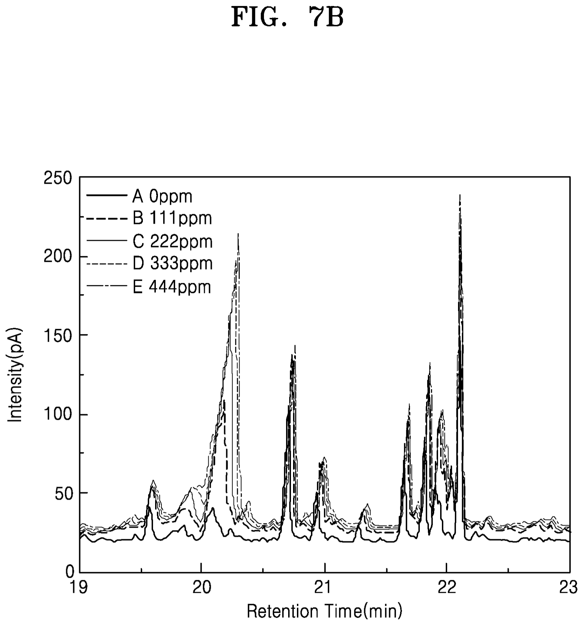

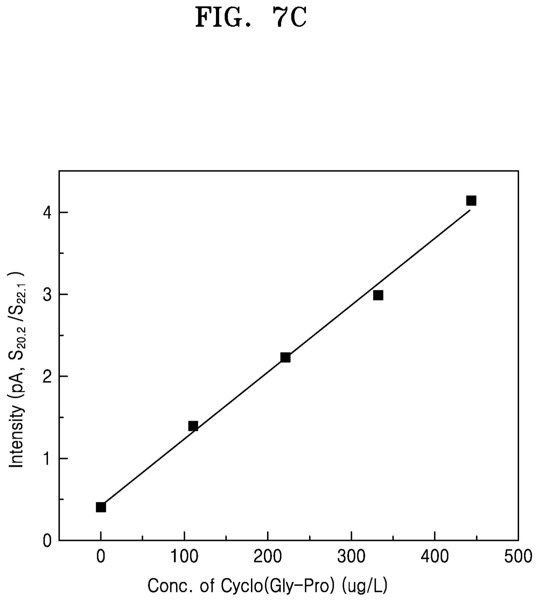

As a result of the analysis, as shown in FIGS. 7A to 7C, it was confirmed that the peak of the Cyclo(Gly-Pro) standard substance was 20.2 minutes, and a calibration curve of Y = 0.0081X + 0.4248 (R2 = 0.996) was obtained. Consequently, the presence of 1.180 ± 0.105 ppm Cyclo(Gly-Pro) in the Strain CX Solution was confirmed through the intersection of the x-axis.

### 3-3. Evaluation of efficacy of Cyclo(Gly-Pro) of alleviating atopic dermatitis

To verify the effect of the Cyclo(Gly-Pro) of alleviating atopic dermatitis, an in vitro efficacy evaluation was conducted.

First, human keratinocytes, HaCaT cells, were purchased from the American Type Culture Collection (ATCC; Manassas, VA, USA) and cultured in DMEM (HyClone, Logan, UT, USA) supplemented with 10 % FBS (HyClone) and 1 % antibiotic-antimycotic solution (Welgene, Daegu, Korea). The cells were maintained at 37 °C in a 5 % CO₂ incubator. After isolating the cells using trypsinization, 4 x 10⁵ cells were added to each well of a 6-well plate. After 1 day of cell attachment, the culture was replaced with a serum-free medium and treated with IFN-γ (Sigma Aldrich, St. St. Louis, MO, USA) at a concentration of 10 ng/mL to induce an environment similar to atopic dermatitis. As a positive control group, 1 µM dexamethasone (Sigma Aldrich) was used. As experimental groups, the cells were treated with Cyclo(Gly-Pro) (Biosynth Ltd, Berkshire, UK) at concentrations of 1, 10, and 100 ppm. After 24 hours, all groups underwent RNA isolation for real-time PCR analysis. The RNA isolation was performed using a RNeasy Mini kit (Qiagen, Valencia, CA), and 1 µg of RNA per sample was synthesized into cDNA using ReverTra Ace^{™} qPCR RT Master Mix (TOYOBO, Japan). The reaction conditions for cDNA synthesis was 90 °C for 15 minutes. The Real-time PCR was performed using PowerSYBR^{®} Green PCR Master Mix (Applied biosystems, Warrington, UK) and the following primer pairs were used.
GAPDH (Forward: 5'-TCAACGGATTTGGTCGTATTGGG-3' (SEQ ID NO: 8); Reverse: 5'-TGATTTTGGAGGGATCTCGC-3' (SEQ ID NO: 9)),
CCL17/TARC (Forward: 5'-TTCTCTGCAGCACATCCACGCA-3' (SEQ ID NO: 10); Reverse: 5'-CTGGAGCAGTCCTCAGATGTCT-3' (SEQ ID NO: 11)),
CCL22/MDC (Forward: 5'-TTACGTCCGTTACCGTCTGC-3' (SEQ ID NO: 12); Reverse: 5'-CCACGGTCATCAGAGTAGGC-3' (SEQ ID NO: 13)),
IL-13 (Forward: 5'-TCAATCCTCTCCTGTTGGCAC-3' (SEQ ID NO: 14); Reverse: 5'-GATGCTCCATACCATGCTGC-3' (SEQ ID NO: 15)),
IL-22 (Forward: 5'-CTCAGCAACAGGCTAAGCACA-3' (SEQ ID NO: 16); Reverse: 5'-CTTTGCTCTGGTCAAATGCAGG-3' (SEQ ID NO: 17)),
sIL-2R (Forward: 5'-AGGGCTCTACACAGAGGTCC-3' (SEQ ID NO: 18); Reverse: 5'-GTGACGAGGCAGGAAGTCTC-3' (SEQ ID NO: 19)),
IL-31 (Forward: 5'-TTGCCCGTCCGTTTACTACG-3' (SEQ ID NO: 20); Reverse: 5'-CTGTGGATGTTGTTTGGCGG-3' (SEQ ID NO: 21)),
FLG (Forward: 5'-TGAAGCCTATGACACCACTGA-3' (SEQ ID NO: 22); Reverse: 5'-TCCCCTACGCTTTCTTGTCCT-3' (SEQ ID NO: 23)).

The cDNA amplification was performed by 40 cycles of amplification, wherein each cycle has the following conditions: 50 °C for 2 minutes, 95 °C for 10 minutes, 40 cycles of 95°C for 10 seconds, and 60°C for 1 minute. GAPDH was used as an ISTD material, and the mRNA expression levels were expressed as relative values compared to the negative control group. The results values are presented as the mean ± standard deviation (SD) from at least three independent experiments.

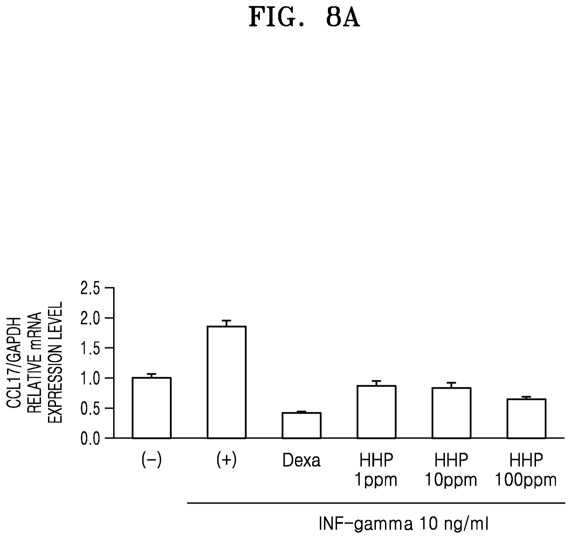

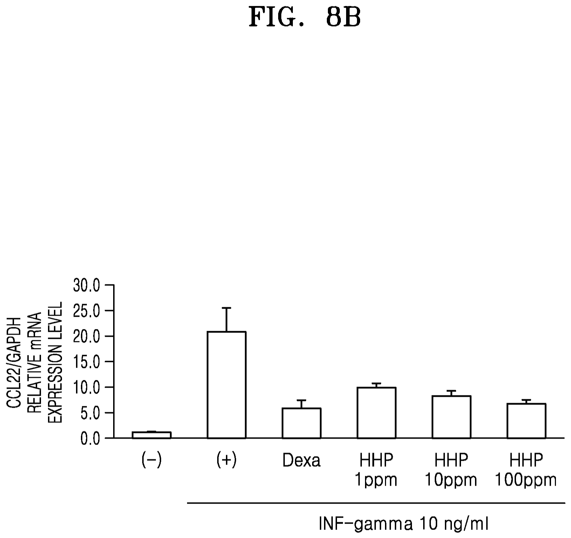

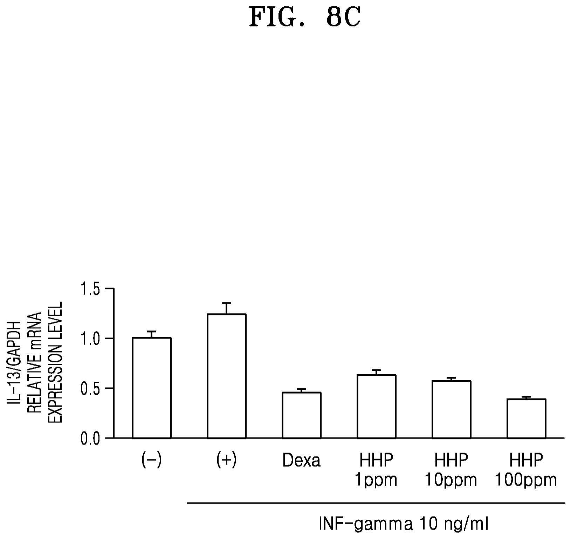

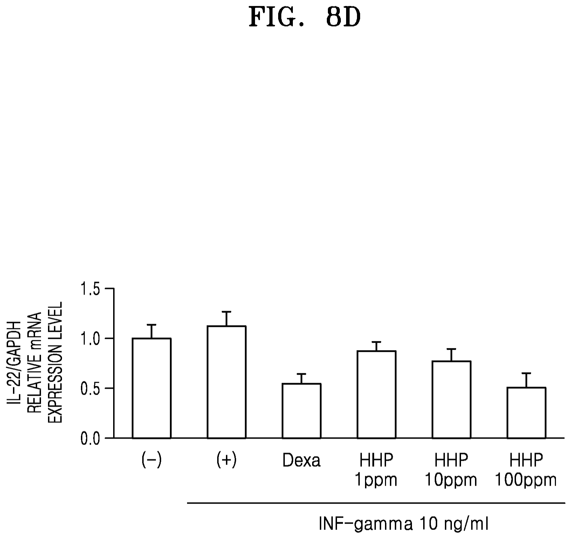

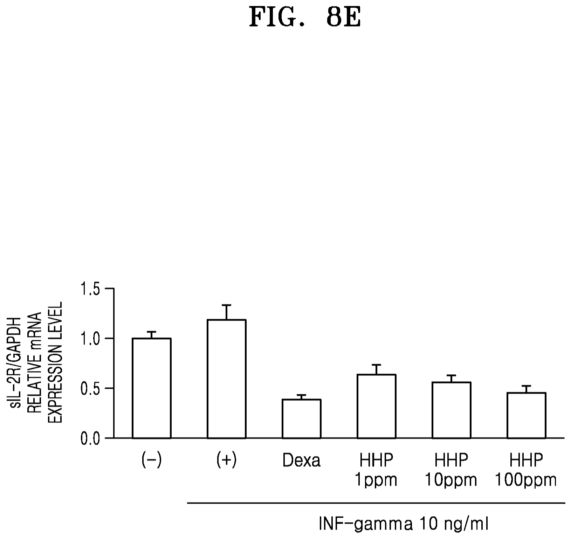

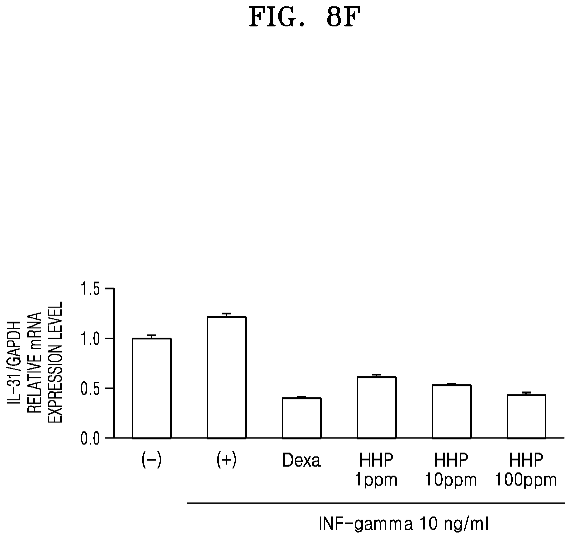

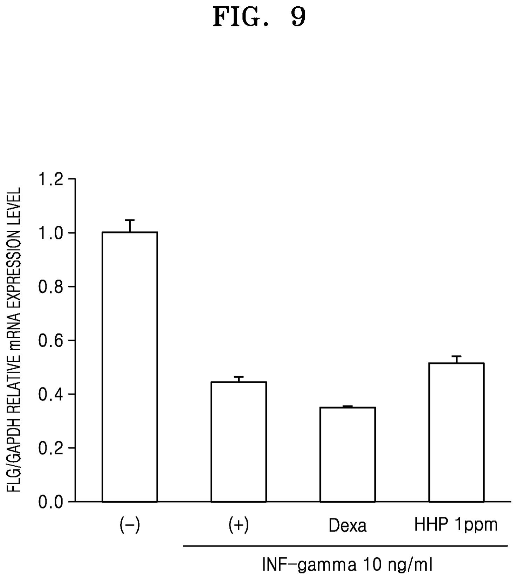

As a result, as shown in FIGS. 8A to 8F and FIG. 9, the atopic dermatitis cell model treated with the Cyclo(Gly-Pro) decreased the relative expression levels of the inflammatory markers CCL17, CCL22, IL-13, IL-22, sIL-2R, and IL-31, which were increased by INF-gamma, and increased the relative expression levels of filaggrin (FLG), which was decreased by INF-gamma.

### Conclusion

As shown in FIG. 1, it was confirmed that the antipruritic efficacy of the streptococcus strain culture filtrate showed a synergistic effect when mixed with the S. *infantis* CX-4.

As shown in FIGS. 2 to 4, it was confirmed that the Streptococcus postbiotic mixture, Strain CX Solution&, according to an embodiment improved the weakened barrier function of the atopic dermatitis patients and alleviated atopic dermatitis symptoms by relieving itchiness.

In addition, as shown in FIG. 5, the Strain CX Solution as the Streptococcus postbiotic mixture according to an embodiment increased the relative abundance of microorganisms of the genus Streptococcus, which are beneficial skin bacteria, and suppressed S, *aureus,* which is a microorganism causing atopy, and accordingly, it was confirmed that the skin microbiome, i.e., the skin microbial community, of the atopic dermatitis patients were improved and normalized.

Additionally, as shown in FIGS. 6 to 9, it was confirmed that the core efficacy substance of the Strain CX Solution for the alleviation of atopic dermatitis is Cyclo(Gly-Pro).

These results indicate that three Streptococcus spp. strains, cultures, lysates, extracts, or fermentation products thereof, or Cyclo(Gly-Pro) thereof as a core efficacy substance can be utilized in the composition for the prevention, alleviation, or treatment of skin disease. These ingredients are expected to be particularly useful in the prevention, alleviation, or treatment of atopic dermatitis, skin inflammatory disease, and itching (pruritus).

### [Accession No]

Name of Depository Authority: Korean Culture Center of Microorganisms (international)
Accession No : KCCM12005P
Accession date : 20170403
Name of Depository Authority: Korean Culture Center of Microorganisms (international)
Accession No : KCCM12642P
Accession date : 20191218
Name of Depository Authority: Korean Culture Center of Microorganisms (international)
Accession No : KCCM12656P
Accession date : 20200115

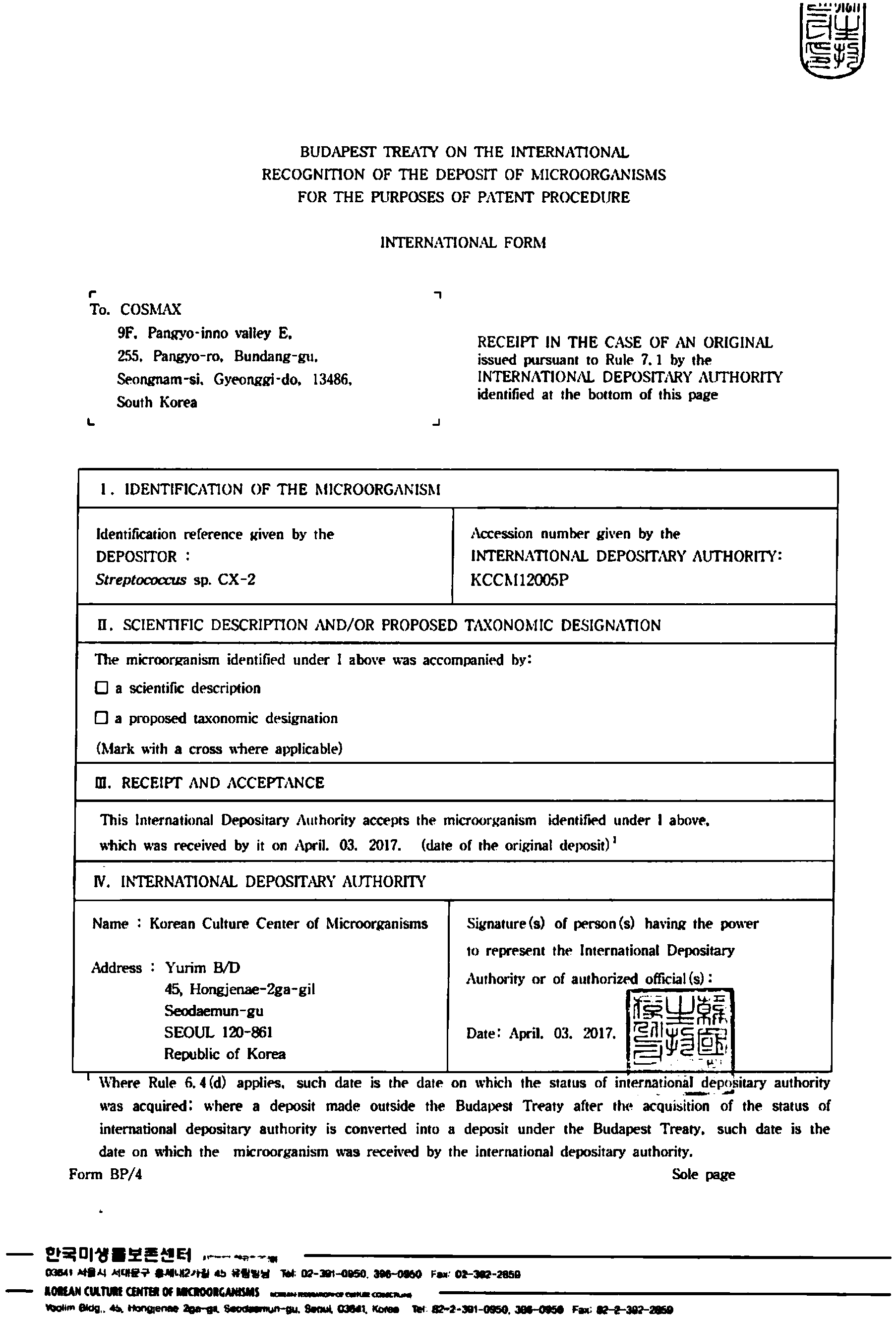
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX
9F. Pangyo-inno valley E.
255. Pangyo-ro. Bundang-gu.
Seongnam-si. Gyeonggi-do. 13486.
South Korea

RECEIPT IN THE CASE OF AN ORIGINAL
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR :
Streptococcus sp. CX-2 | Accession number given by the
INTERNATIONAL DEPOSITARY AUTHORITY:
KCCM12005P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 03. 2017. (date of the original deposit)¹
IV. INTERNATIONAL DEPOSITARY AUTHORITY
Name : Korean Culture Center of Microorganisms

Address : Yurim B/D
45, Hongjenae-2ga-gil
Seodaemun-gu
SEOUL 120-861
Republic of Korea | Signature(s) of person(s) having the power
to represent the International Depositary
Authority or of authorized official(s):

Date: April. 03. 2017.
¹ Where Rule 6.4(d) applies, such date is the date on which the status of international depositary authority
was acquired; where a deposit made outside the Budapest Treaty after the acquisition of the status of
international depositary authority is converted into a deposit under the Budapest Treaty, such date is the
date on which the microorganism was received by the international depositary authority.
Form BP/4 Sole page

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS [?]
Yoolim Bldg., 45, Hongjenae 2ga-gil, Seodaemun-gu, Seoul, 03641, Korea Tel: 82-2-391-0950, 396-0950 Fax: 82-2-392-2859

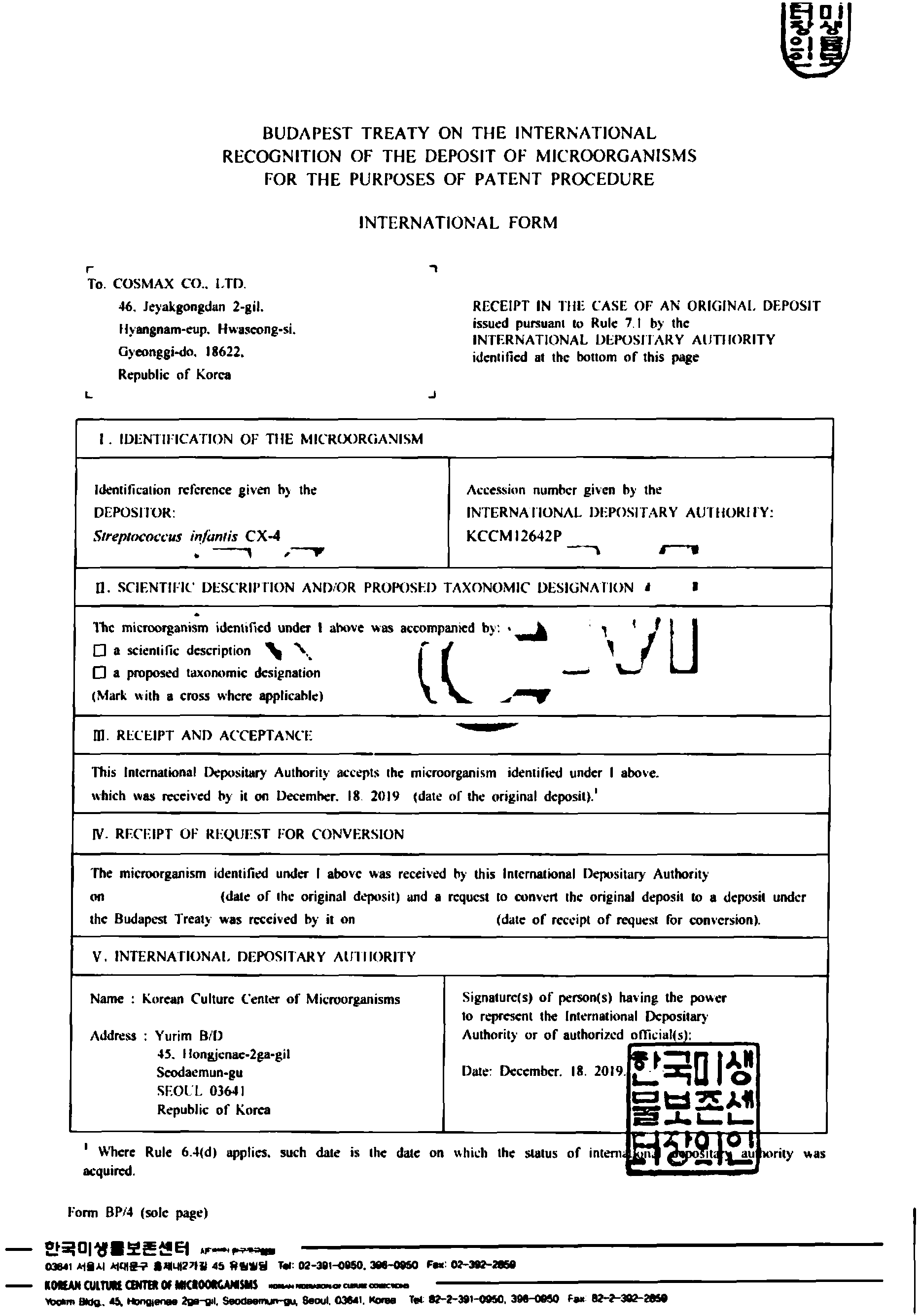

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX CO., LTD.
46, Jeyakgongdan 2-gil,
Hyangnam-eup, Hwaseong-si,
Gyeonggi-do, 18622,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Streptococcus infantis CX-4 | Accession number given by the
INTERNATIONAL DEPOSITARY AUTHORITY:
KCCM12642P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 18. 2019 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 18. 2019.


¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS [?]
Yoorim Bldg., 45, Hongjenae 2ga-gil, Seodaemun-gu, Seoul, 03641, Korea Tel: 82-2-391-0950, 396-0950 Fax: 82-2-392-2859

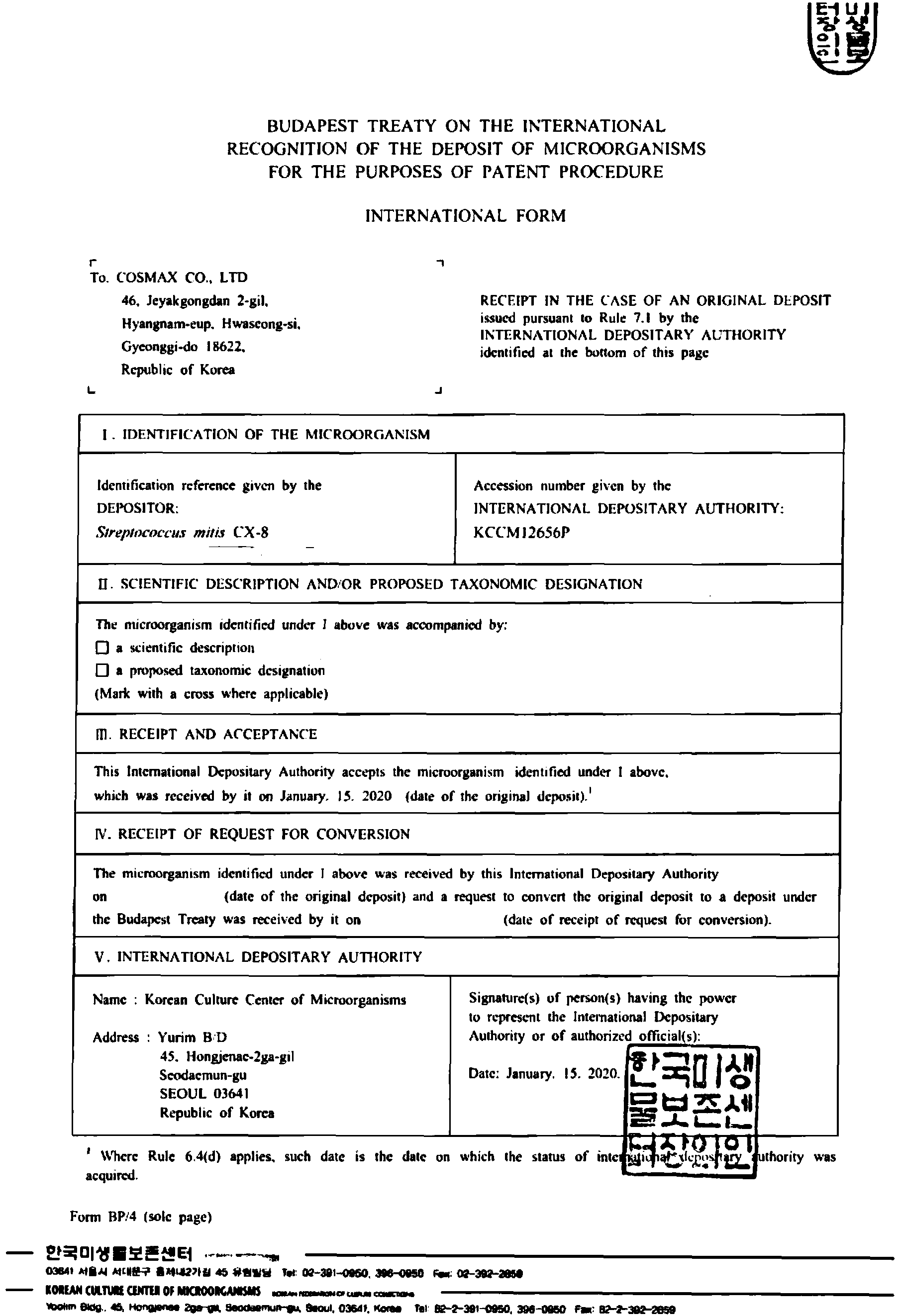
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX CO., LTD
46, Jeyakgongdan 2-gil,
Hyangnam-eup, Hwaseong-si,
Gyeonggi-do 18622,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Streptococcus mitis CX-8 | Accession number given by the
INTERNATIONAL DEPOSITARY AUTHORITY:
KCCM12656P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on January. 15. 2020 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 15. 2020.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A cosmetic composition for prevention or alleviation of skin disease, comprising a mixture of (1), (2), and (3) below:
(1) a *Streptococcus pneumoniae* strain, a culture, a lysate, an extract, or a fermentation product thereof;
(2) a *Streptococcus infantis* strain, a culture, a lysate, an extract, or a fermentation product thereof; and
(3) a *Streptococcus mitis* strain, a culture, a lysate, an extract, or a fermentation product thereof.

2. The cosmetic composition of claim 1, wherein the mixture contains Cyclo(Gly-Pro) derived from the strains.

3. The cosmetic composition of claim 1, wherein the *Streptococcus pneumoniae* strain is a *Streptococcus* CX-2 strain deposited under accession number KCCM12005P,
the *Streptococcus infantis* strain is a *Streptococcus infantis* CX-4 strain deposited under accession number KCCM12642P, and
the *Streptococcus mitis* strain is a *Streptococcus mitis* CX-8 strain deposited under accession number KCCM12656P.

4. The cosmetic composition of claim 1, wherein the skin disease is at least one selected from the group consisting of atopic dermatitis, contact dermatitis, seborrhea, acne vulgaris, and pruritus.

5. A cosmetic composition for prevention or alleviation of skin disease, comprising Cyclo(Gly-Pro) as an active ingredient.

6. A pharmaceutical composition for prevention or treatment of skin disease, comprising a mixture of (1), (2), and (3) below:
(1) a *Streptococcus pneumoniae* strain, a culture, a lysate, an extract, or a fermentation product thereof;
(2) a *Streptococcus infantis* strain, a culture, a lysate, an extract, or a fermentation product thereof; and
(3) a *Streptococcus mitis* strain, a culture, a lysate, an extract, or a fermentation product thereof .

7. The pharmaceutical composition of claim 6, wherein the mixture contains Cyclo(Gly-Pro) derived from the strains.

8. The pharmaceutical composition of claim 6, wherein the *Streptococcus pneumoniae* strain is a *Streptococcus* CX-2 strain deposited under accession number KCCM12005P,
the *Streptococcus infantis* strain is a *Streptococcus infantis* CX-4 strain deposited under accession number KCCM12642P, and
the *Streptococcus mitis* strain is a *Streptococcus mitis* CX-8 strain deposited under accession number KCCM12656P.

9. The pharmaceutical composition of claim 6, wherein the skin disease is at least one selected from the group consisting of atopic dermatitis, contact dermatitis, seborrhea dermatitis, acne vulgaris, and pruritus.

10. A pharmaceutical composition for prevention or treatment of skin disease, comprising Cyclo(Gly-Pro) as an active ingredient.
